# EUROPÄISCHE PATENTSCHRIFT

(11) **EP 1 659 109 B1**
(45) Veröffentlichungstag und Bekanntmachung des Hinweises auf die Patenterteilung: **21.03.2007**
(21) Anmeldenummer: 05024047.2
(22) Anmeldetag: 04.11.2005
(51) Int. Cl.: C07C 231/12, C07C 233/36

(54) **Verfahren zur Herstellung hochkonzentrierter fliessfähiger wässriger Lösungen von Betainen**
Process for the preparation of highly concentrated, flowable aqueous betaine solutions
Procédé pour la préparation de solutions fluides à haute concentration aqueuse de betaines

(30) Priorität: 17.11.2004 DE 102004055549
(43) Veröffentlichungstag der Anmeldung: 24.05.2006
(73) Patentinhaber: Goldschmidt GmbH, 45127 Essen (DE)
(72) Erfinder: Allef, Petra, 53121 Bonn (DE); Begoihn, Uwe, 45359 Essen (DE); Grüning, Burghard, 45134 Essen (DE); Klein, Ralf, 42549 Velbert (DE); Peggau, Jörg, 45357 Essen (DE)

(56) Entgegenhaltungen:
- EP-A- 0 302 329
- EP-A- 0 353 580
- DE-A1- 10 019 142

## Beschreibung

Die Erfindung betrifft ein Verfahren zur Herstellung hochkonzentrierter fließfähiger wässriger Lösungen von Betainen, mit Betaingehalten von bis zu ca. 55 Gew.-%.

Betaine haben sich in den letzten Jahren in der kosmetischen Industrie als fester Bestandteil von Rezepturen, insbesondere für die Haar- und Körperreinigung, etabliert. Sie haben die Fähigkeit, einen dichten und sahnigen Schaum auszubilden, der auch in Gegenwart anderer Tenside, Seifen und Additive über einen langen Zeitraum stabil bleibt, verbunden mit anerkannt guten Reinigungseigenschaften ohne irgendwelche irritierenden Nebenwirkungen, selbst für empfindliche Haut.

Die Herstellung von Betainen wird in der einschlägigen Patent- und Fachliteratur ausführlich beschrieben (US-3 225 074). Im Allgemeinen werden dabei tertiäre Aminstickstoffatome enthaltende Verbindungen mit ω-Halogencarbonsäuren oder deren Salzen in wässrigen oder wasserhaltigen Medien umgesetzt.

Als tertiäre Aminstickstoffatome enthaltende Verbindungen werden insbesondere Fettsäureamide der allgemeinen Formel (I)

R³-CONH-(CH₂)ₘ-NR⁴R⁵ (I)

eingesetzt, worin
- R³: der Alkylrest einer Fettsäure ist, der gegebenenfalls verzweigt, gegebenenfalls Mehrfachbindungen, gegebenenfalls Hydroxylgruppen enthalten kann,
- R⁴, R⁵: gleiche oder verschiedene Alkylreste mit 1 bis 4 C-Atomen bedeuten und
- m: 1 bis 3 sein kann.

Der Alkylrest R³ kann sich hierbei von den natürlichen oder synthetischen Fettsäuren mit 6 bis 20 C-Atomen, vorzugsweise von den natürlichen pflanzlichen oder tierischen Fettsäuren mit 8 bis 18 C-Atomen ableiten sowie deren natürlich vorkommenden oder speziell eingestellten Mischungen miteinander oder untereinander.

Als Fettsäuren kommen beispielsweise Capronsäure, Caprylsäure, Caprinsäure, Laurinsäure, Palmitinsäure, Stearinsäure, Behensäure, Linolsäure, Linolensäure, Ricinolsäure in Betracht.

Bevorzugt werden die natürlich vorkommenden Fettsäuremischungen mit einer Kettenlänge von 8 bis 18 C-Atomen, wie Kokosfettsäure oder Palmkernfettsäure, welche gegebenenfalls durch geeignete Hydrierungsmethoden gehärtet werden können.

Diese Fettsäuren bzw. Fettsäuremischungen werden durch übliche Kondensationsreaktion in einer ersten Verfahrensstufe bei 140 bis 200 °C mit Aminen der allgemeinen Formel (II)

H₂N-(CH₂)ₘ-NR⁴R⁵ (II)

- in welcher R⁴ und R⁵ und m die in der Formel (I) genannte Bedeutung haben - zu den Fettsäureamiden mit tertiären Stickstoffatomen der allgemeinen Formel (I) umgesetzt.

Die anschließende Quaternierungsreaktion zu Betainen der Formel (III)

R³-CONH-(CH₂)ₘ-N⁺R⁴R⁵(CH₂)_{y}COO⁻ (III)

worin R³, R⁴, R⁵ und m die gleiche Bedeutung wie in den Formeln (I) und (II) haben und y = 1, 2, 3 sein kann, wird nach den literaturbekannten Verfahren als 2. Verfahrensstufe durchgeführt.

Dem Fettsäureamid der Formel (I) werden dabei in der Regel im wässrigen Medium ω-Halogenalkylcarbonsäuren, vorzugsweise Chloressigsäure oder das Natriumsalz der Chloressigsäure, zugesetzt und bei einer mehrstündigen Reaktion bei ca. 80 bis 100 °C die Quaternierung vollzogen. Je nach eingesetzter Fettsäure bzw. Fettsäuremischung muss zum Erhalt der Rührfähigkeit bei fortschreitender Reaktion eine Mindestmenge an Wasser vorhanden sein. Die handelsübliche Feststoffkonzentration der auf diese Weise hergestellten Betain-Lösungen liegt daher bei etwa 35 bis 37 Gew.-% oder darunter.

Zur Einsparung von Lagerungs- und Transportkosten sowie aus formulierungstechnischen Gründen bei der Weiterverarbeitung ist aber in vielen Fällen eine höhere Konzentration dringend erwünscht. In der Vergangenheit sind daher eine Reihe von Verfahren zur Lösung dieser Anforderung vorgeschlagen worden. Der Feststoffgehalt dieser meist hochviskosen Lösungen konnte jedoch nur auf ca. 50 % erhöht werden, bei kommerziellen hochkonzentrierten Betainen liegt er meist bei ca. 45 %, der aktive Betaingehalt damit bei etwa 35 %.

Aus der DE-C-3 613 944 ist ein Verfahren bekannt, bei dem die Quaternierung in einem organischen polaren Lösungsmittel mit einem Wasseranteil von 20 Gew.-% durchgeführt und danach das Lösungsmittel ganz oder teilweise destillativ entfernt und dann wieder mit einem anwendungstechnisch brauchbaren Lösungsmittel auf die gewünschte Konzentration eingestellt wird. Abgesehen davon, dass das Verfahren technisch aufwendig und kostenintensiv ist, sind organische Lösungsmittel bzw. auch die destillativ nicht entfernbaren Restgehalte bei der Weiterverarbeitung in kosmetischen Rezepturen vielfach unerwünscht.

Das Verfahren gemäß DE-C-3 826 654 enthält zwar ein Beispiel ohne organisches Lösungsmittel, jedoch sind die erhaltenen Viskositäten für eine großtechnische Produktion zu hoch. Außerdem werden größere Mengen (3 bis 20 Gew.-%) eines nicht-ionogenen Tensides (Polyoxyethylenether mit 10 bis 250 Oxyethyleneinheiten) als Verflüssiger benötigt. Zusatzmengen in dieser Größenordnung sind unerwünscht, da sie die Endformulierungen physikalisch und/oder physiologisch negativ beeinträchtigen können.

DE-C-4 207 386 beschreibt hochkonzentrierte Betaine, die 1 bis 3 Gew.-% gesättigte oder ungesättigte Fettsäure als verdünnendes Prinzip enthalten. Betaine, die nach dieser Methode hergestellt werden, können bis zu einem Feststoffgehalt von 48 Gew.-% aufkonzentriert werden.

Ähnliche Konzentrationen werden nach EP-B-1 140 798 erhalten. Als verdünnendes Prinzip dienen hier Glutaminsäure(salze) und analoge Aminosäuren, die der Quaternisierungsreaktion zugesetzt werden.

DE-C-19 505 196 beschreibt die Verwendung von Sulfobetainen, Amphoglycinaten, Trimethylglycin oder Dicarbonsäure(diamiden) in Konzentrationen von 4 bis 8 Gew.-% während der Carboxymethylierung. Die erhaltenen Betaine haben einen Feststoffgehalt von ca. 50 Gew.-% und eine mittlere Viskosität.

Ebenfalls 50 Gew.-% Feststoffgehalt haben Betaine, die nach DE-A-19 700 798 unter Verwendung von bis zu 5 Gew.-% mehrwertigen ggf. hydroxyfunktionalisierten Carbonsäuren während der Carboxymethylierung hergestellt wurden.

Ähnliche Feststoffgehalte erreicht man durch Zusatz von 0,05 bis 2 Gew.-% Cyclodextrinen oder Dextranen, wie in DE-A-10 207 924 beschrieben.

Der Einsatz von 0,5 bis 5 Gew.-%, vorzugsweise 2 bis 4 Gew.-% Betainen aus kurzkettigen Mono- oder Dicarbonsäuren zur Verdünnung von Kokosbetain wird in EP-C-656 346 beschrieben. Dabei werden Betaine mit einer Feststoffkonzentration von 45 Gew.-% erhalten.

DE-C-4 408 183 beschreibt Betaine mit bis zu 54 Gew.-% Festkörper. Sie enhalten 1 bis 10 Gew.-% einer Hydroxycarbonsäure. Um fließfähige Produkte bei über 50 Gew.-% Festkörper zu erhalten sind ca. 5 Gew.-% Zitronensäure nötig. Der Betaingehalt beträgt dann ca. 32 Gew.-%.

DE-C-19 523 477 beschreibt die Verwendung von mehrwertigen Carbonsäureamiden, z.B. Adipinsäurediamidamin, während der Carboxymethylierung. Die beschrieben Betainkonzentrate enthalten zwar 60 Gew.-% Festkörper, sind aber nicht mehr dünnflüssig. Da während der Carboxymethylierung ein Viskositätsmaximum durchlaufen wird, ist eine großtechnische Herstellung dieser Produkte sehr aufwendig und kompliziert, es sei denn große Mengen an Adipinsäurebetain werden dem Betain zugesetzt, was wiederum die Eigenschaften des Betains stark beeinflusst.

Feststoffgehalte von ca. 50 Gew.-% werden auch nach US-B-6 683 033 erhalten. Dabei werden 0,5 bis 3,5 Gew.-% Phosphorsäureester von ggf. niedrig ethoxylierten Fettalkoholen und/oder Dimersäuren als Verflüssiger verwendet. Der Betaingehalt der Beispiele liegt unter 37 %.

DE-C-4 408 228 beschreibt Betaine mit Feststoffgehalte über 50 Gew.-%, enthaltend 1 bis 10 Gew.-% eines nichtionischen Tensides mit HLB 6 bis 12 und/oder Hydroxycarbonsäuren, 1 bis 6 Gew.-% Polyole und ggf. 1 bis 10 Gew.-% Fettsäure(salze). Für hohe Feststoffgehalte sind auch hier meist > 6 Gew.-% Zusatzstoffe nötig, was im Hinblick auf potentiell negative Einflüsse auf Formulierungen nicht wünschenswert ist. Der eigentliche Betaingehalt liegt bei nur ca. 32 Gew.-%.

Aufgrund der im Vergleich zum Produktpreis hohen Lager- und Transportkosten besteht ein Bedarf an noch höher konzentrierten, fließ- und pumpfähigen, wässrigen Lösungen von Betainen, welche frei von niedrigen Alkoholen wie Methanol, Ethanol, Propanol oder Isopropanol sind.

Aufgabe der vorliegenden Erfindung war es daher, ein Verfahren zur Herstellung eines fließ- und pumpfähigen Betainkonzentrates bereitzustellen, mit dem wesentlich höhere Feststkörper-/Aktivsubstanz-Gehalte erzielbar sind als mit den bekannten Verfahren wobei die Reaktionsgemische während des gesamten Produktionsverfahrens gut handhabbar und nicht zu hochviskos sind und die Reaktionsprodukte bei späterer Lagerung nicht nachdicken oder gelieren.

Es ist literaturbekannt (z.B. EP-A-679 180 und Referenzen darin), dass bei herkömmlicher Herstellung von Tensidkonzentraten, sich erst Kugel-Mizellen bilden, die dann in Stäbchen-Mizellen übergehen. Dieser Zustand wird allgemein als M₁-Phase bezeichnet.

In Tensidgemischen mit höherer Konzentration können sich flüssigkristalline Strukturen, sogenannte lamellare Phasen oder G-Phasen, bilden. Diese werden meist in einem engen Bereich zwischen 40 und 85 Gew.-% Feststoffgehalt gebildet. Die G-Phase ist eine pumpbare Flüssigkeit, in der die Tensidmoleküle sich in Lagen, so genannten lamellaren Schichten anordnen.

In EP-A-679 180 werden zur Herstellung solcher G-Phasen 5 bis 45 Gew.-% eines nicht wassser-mischbaren organischen Lösungsmittels verwendet, in DE-A-2 921 366 5 bis 45 Gew.-% nichtionischen Tensides mit einem HLB-Wert im Bereich 6 bis 12.

Überraschenderweise wurde nun gefunden, dass man fließ- und pumpfähige Betain-Lösungen mit Betaingehalten von > ca. 32 insbesondere 35 Gew.-% und bis zu ca. 55 Gew.-% und höher aus dem Reaktionsgemisch aus Fettsäureamid und ω-Halogenalkylcarbonsäure nach bekannten Verfahren herstellen kann, wenn man vor oder während der Quaternierungsreaktion kleine Mengen von 0,1 bis < 3 Gew.-%, vorzugsweise 0,1 bis 2 Gew.-%, insbesondere 0,3 bis 1 Gew.-%, bezogen auf das Endprodukt, eines oder mehrerer mizellarer Verdicker, vorzugsweise eines oder mehrerer nichtionogener, vorzugsweise hochethoxylierter Tenside zusetzt.

Die Feststoffgehalte, bestehend aus dem Betaingehalt und dem Gehalt an weiteren nichtflüchtigen Reaktionsnebenprodukten liegen entsprechend den jeweiligen Herstellungsverfahren (Reaktionsparameter, molare Verhältnisse) und möglichen zusätzlichen Komponenten im Bereich von ca. 40 bis ca. 70 Gew.-%.

Diese zugesetzten Tenside verhindern wirkungsvoll den während der Quarternisierung auftretenden Viskositätsanstieg ganz oder teilweise und erlauben so die Herstellung von hochkonzentrierten, gelfreien Betainlösungen, die aufgrund der ebenfalls reduzierten Viskosität der Endprodukte fließfähig und pumpbar sind.

Ein Gegenstand der Erfindung ist daher ein Verfahren zur Herstellung hochkonzentrierter fließ- und pumpfähiger wässriger Lösungen von Betainen der allgemeinen Formel (III)

R³-CONH-(CH₂)ₘ-N⁺R⁴R⁵(CH₂)_{y}COO⁻ (III)

worin
- R³: der Alkylrest einer Fettsäure ist, der gegebenenfalls verzweigt, gegebenenfalls Mehrfachbindungen, gegebenenfalls Hydroxylgruppen enthalten kann,
- R⁴, R⁵: gleich oder verschiedene Alkylreste mit 1 bis 4 C-Atomen bedeuten,
- m: 1 bis 3 und
- y: 1, 2, 3 sein kann,
mit einem Betaingehalt von mindestens 32 Gew.-%, vorzugsweise mindestens 35 Gew.-%, durch Quaternierung von tertiären Aminstickstoff enthaltenden Verbindungen mit ω-Halogencarbonsäuren nach bekannten Verfahren, welches dadurch gekennzeichnet ist, dass der Reaktionsmischung vor oder während der Quaternierungsreaktion 0,1 bis < 3 Gew.-%, vorzugsweise 0,1 bis 2 Gew.-%, insbesondere 0,3 bis 1 Gew.-%, bezogen auf das Endprodukt, eines oder mehrerer mizellarer Verdicker, insbesondere wasserlösliche, nichtionische Tenside, vorzugsweise hochethoxliert, d.h. mit einem HLB > 12, zugesetzt werden.

Die erfindungsgemäße Lehre kann synergistisch zu allen bekannten Verdünnungsprinzipien angewendet werden. Im Allgemeinen werden bis zu 20 Gew.-% höhere Betaingehalte erhalten als durch die literaturbekannten Verfahren allein. Bei höheren Anteilen an mizellaren Verdickern, insbesondere > ca. 1 bis 1,5 bis < 3 Gew.-%, werden diese vorzugsweise in Mischung untereinander und/oder mit den aus dem Stand der Technik bekannten Verflüssigungsmitteln eingesetzt.

Ein weiterer Gegenstand der Erfindung ist daher ein Verfahren zur Herstellung hochkonzentrierter fließ- und pumpfähiger wässriger Lösungen von Betainen durch Quaternierung von tertiären Aminstickstoff enthaltenden Verbindungen mit ω-Halogencarbonsäuren nach bekannten Verfahren, welches dadurch gekennzeichnet ist, dass der Reaktionsmischung vor, während oder nach der Quaternierungsreaktion zusätzlich zu den mizellaren Verdickern mindestens eine Verbindung ausgesucht aus der Gruppe der Sulfobetaine, Amphoglycinate, Trimethylglycine, Aminosäuren, Monocarbonsäureamide, Monocarbonsäureamidoamine, Di-/Polycarbonsäuremono- und/oder -diamide, einwertigen, gegebenenfalls hydoxyfunktionalisierten Carbonsäuren, di-/polyfunktionellen, gegebenenfalls hydoxyfunktionalisierten Carbonsäuren, wasserlöslichen Kohlenhydraten, Polyole in den üblichen literaturbekannten Konzentrationen zugesetzt wird.

Es war überraschend, dass diese geringen Mengen an mizellaren Verdickern diesen sprunghaft verbesserten Effekt bei höher konzentrierten Lösungen aufweisen, da gemäß der Lehre der DE-C-3 826 654 erst der Zusatz größerer Mengen > 3 % nichtionogener Tenside einen viskositätserniedrigenden Effekt bewirken soll.

Überraschend war auch die Erkenntnis, dass vor allem bei lösungsmittelfreien Arbeiten und Feststoffgehalten > 45 Gew.-%. der Zusatz von > 3 Gew.-% wie in DE-C-3 826 654 beschrieben, meist zu einem gegenteiligen Effekt, d.h. zu einer Erhöhung der Viskosität führt.

Mizellare Verdicker im Sinne der vorliegenden Erfindung sind grenzflächenaktive Verbindungen, die häufig zur Erhöhung der Viskosität von tensidhaltigen Formulierungen eingesetzt werden, sie werden üblicherweise auch als assoziative Verdicker bezeichnet. Man kann hydrophile und hydrophobe Verdicker unterscheiden.

Geeignete mizellare Verdicker bestehen vorzugsweise aus einem oder mehreren oligomeren oder polymeren hydrophilen Grundbausteinen vorzugsweise ausgewählt aus den Produktgruppen der Polyalkylenoxide, Polyglycidol, Polyglycerin oder Polyamine verbunden mit langkettigen lipophilen Alkylresten.

Geeignete hydrophile mizellare Verdicker sind Anlagerungsprodukte von Alkylenoxiden an geeignete Startermoleküle wie Wasser, Alkohole, Säuren, Amine mit gegebenenfalLs veresterten oder veretherten Endgruppen.

Als Alkylenoxide werden insbesondere Ethylenoxid oder Propylenoxid zur Bildung von Homopolymeren oder Polymeren mit statistischer oder blockweiser Verteilung verwendet. Diese Polymeren können weiterhin durch Addition von Alkylenoxiden an vorzugsweise niedermolekulare, ein- oder mehrwertige Alkohole oder Amine wie Methanol, Ethanol, Butanol, Pentanol und deren höhere Homologe, Ethylen- und Polyethylenglycole, Propylen- und Polypropylenglycole und deren höhere Homologe, Glycerin, Polyglycerine, Zuckeralkohole, Mono/Dialkyl-ethanolamine, Mono/Di-isopropanolamine, Polyamine wie insbesondere alkylsubstituiertes Diaminoethan, Diaminopropan, Diethylentriamin, Triethylentetramin und die jeweiligen höheren Homologen hergestellt werden.

Die langkettigen lipophilen Alkylreste leiten sich ab von Carbonsäuren, vorzugsweise Fettsäuren, Fettalkoholen oder Fettaminen. Die Verknüpfung der hydrophilen und lipophilen Grundbausteine kann z. B. durch Veresterungsreaktionen oder Etherbildung, wie Addition an Fettsäuren, Alkoholen oder Aminen erfolgen.

Als Säuren kommen ein- oder mehrwertige monomere oder polymere organische Säuren in Frage wie die homologe Reihe der Mono- und Dicarbonsäuren, insbesondere beispielsweise Palmitinsäure, Stearinsäure, Ölsäure, Ricinolsäure, Hydroxystearinsäure, Adipinsäure, Sebazinsäure, Azelainsäure, dimere Fettsäure.

Als Amine sind ein- oder mehrwertige, monomere oder polymere Verbindungen verwendbar wie beispielsweise die homologe Reihe der Alkanamine wie insbesondere Laurylamin, Stearylamin.

Geeignete Beispiele für mizellare Verdicker sind insbesondere PEG-200 Hydrogenated Glyceryl Palmate, PEG-80 Glyceryl Cocoate, PEG-55 Propylene Glycol Dioleate, PEG-120 Methyl Glucose Dioleate, PEG-200 Hydrogenated Glyceryl Cocoate, PEG-200 Hydrogenated Castor Oil, PEG-30 Glyceryl Cocoate, Block-Copolymerisate von Alkoholen die auch unter dem Namen Pluronic-Typen oder auch Pluriol-Typen bekannt sind.

Weitere Beispiele sind PEG-150 Distearate, PEG-100 Stearate, PEG-40 Stearate.

Als amingestartete Verbindungen sind Ethylendiamin-Blockpolymere, alkoxylierte Amine und Polyamine geeignet.

Weitere nichtpolymere, eher hydrophobe mizellare Verdicker können z.B. die folgenden Strukturen aufweisen: Cocamide DEA (Amid der Kokosfettsäure mit Diethanolamin), Oleamide DEA, Isostearamide MIPA (= Methylisopropanolamin), Glyceryl Laurate, Glyceryl Oleate, Polyglyceryl-3-Caprate, Laureth-4, Palmamidopropyltrimonium Chloride, Bis (oleylcarboxyethyl)-hydroxyethyl-methylammonium-methosulfate.

Für die erfindungsgemäßen Systeme zeigen die Strukturen der hydrophilen Verdicker mit einem Molekulargewicht von größer als 1.500 g/mol, vorzugsweise größer als 3.000 g/mol bis ca. 15.000 g/mol die besten Effekte, d.h. in geringen Konzentrationen verhindern sie wirkungsvoll die Bildung einer viskosen Phase und verbessern somit das Fließverhalten hochkonzentrierter Tensidsysteme eindeutig.

Durch den erfindungsmäßigen Zusatz der mizellaren Verdicker wird die Betainlösung bezüglich ihrer Verarbeitungseigenschaften mehrfach verbessert: Die Betainlösungen sind überraschend dünnflüssig, so dass auch in hochkonzentrierten Formulierungen der Gehalt an Lösungsmitteln wie Ethanol merklich reduziert oder ganz vermieden werden kann; sie lassen sich leichter mit Wasser verdünnen, sind kältestabil und trüben auch bei -15 °C nicht ein.

Vorteilhaft für das erfindungsgemäße Verfahren ist, wenn die Amidierung der ersten Stufe in Gegenwart von mindestens einer ein- oder mehrwertigen, gegebenenfalls Mehrfachbindungen und/oder OH-Gruppen enthaltenden Carbonsäuren, insbesondere C₁₋₅-Monocarbonsäuren wie Ameisensäure, Essigsäure, Milchsäure, Acrylsäure, Methacrylsäure, Sorbinsäure und/oder C₂₋₁₀-Di-/Polycarbonsäuren wie Oxalsäure, Malonsäure, Bernsteinsäure, Adipinsäure, Azelainsäure, Malein-(Fumar-)säure durchgeführt wird.

Die Quaternierungsreaktion der Verbindung (I) mit Chloressigsäure und Alkali-/Erdalkalihydroxid bzw. mit dem Alkali-/Erdalkalisalz der Chloressigsäure wird durch mehrstündige Umsetzung im wässrigen Medium bei 80 bis 100 °C durchgeführt, wobei als Viskositätsregulatoren vor oder während der Quaternierungsreaktion 0,1 bis < 3 Gew.-%, vorzugsweise 0,1 bis 1 Gew.-%, eines oder mehrerer wasserlöslicher, nichtionischer Tenside zugesetzt werden.

Als wasserlösliches, nichtionogenes Tensid wird mindestens eine Verbindung ausgesucht aus der Gruppe der Polyoxyethylenester von Fettsäuren oder der Polyoxyethylenether von ggf. partiellen Fettsäureestern mehrwertiger Alkohole, wie Glycerin, Sorbit oder Glucose mitverwendet. Die Polyoxyethylenderivate können auch Anteile von Polyoxypropylen enthalten.

Die zur Herstellung dieser nichtionogenen Tenside verwendeten Fettsäuren sind vorzugsweise die in der Natur vorkommenden Fettsäuren. Sie können gesättigt oder ungesättigt sein. Besonders bevorzugt sind Derivate von Hydroxyfettsäuren, Ricinolsäure oder Hydroxystearinsäure, sowie Glyceride hiervon, z.B. Ricinusöl.

Die Polyoxyethylenether von Fettalkoholen basieren auf Fettalkoholen, die gesättigt oder ungesättigt, substituiert oder unsubstituiert sein können. Beispiele solcher Fettalkohole sind der Lauryl-, Stearyl- und Oleyloalkohol.

Vorzugsweise soll der HLB-Wert dieser nichtionogenen Tenside > 12 liegen. Besonders geeignet sind Polyoxyethylenether und -ester von Hydroxyfettsäuren und Hydroxyfettsäureglyceriden mit einem durchschnittlichen Gehalt von 10 bis 250 Oxyethyleneinheiten.

Ohne die Erfindung darauf einschränken zu wollen, können folgende Beispiele für die erfindungsmäßigen mizellaren Verdicker genannt werden: REWODERM LI 52 (PEG-200 Hydrogenated Glyceryl Palmate), REWODERM LI S 80 bzw. ANTIL 200 (PEG-200 Hydrogenated Glyceryl Palmate), ANTIL 141 (PEG-55 Propylene Glycol Oleate), ANTIL 120 (PEG-120 Methyl Glucose Dioleate), Varonic LI 520 (PEG-200 Hydrogenated Glyceryl Cocoate), REWOPAL PEG 6000 DS (PEG-150 Distearate), TEGO Alkanol S 100 (PEG-100 Stearate) und TAGAT R 200(PEG-200 Hydrogenated Castor Oil).

Die Durchführung des Verfahrens lehnt sich an die im Stand der Technik bekannten Verfahren an, wobei die wesentliche Änderung im Zusatz der als mizellare Verdicker mitverwendeten nichtionogenen Tenside vor oder während der Quaternierungsreaktion besteht. Dabei könnnen alle literaturbekannten Verfahren zur Herstellung konzentrierter Betainlösungen, z.B. Zusatz von ggf. mehrwertigen Hydroxycarbonsäuren, von Polyolen, von Aminosäuren, von kurzkettigen Betainen oder Betainen aus Dicarbonsäuren, additiv angewendet werden. Es werden synergistische Effekte beobachtet. Großtechnisch sind bis zu ca. 20 Gew.-% höherkonzentrierte Betainlösungen erhältlich als in Verfahren, die auf den Einsatz der beschriebenen nichtionogenen Tenside verzichten.

Erfindungsgemäß bevorzugt wird so verfahren, dass die vorgelegte ω-Halogencarbonsäure mit mindestens einem Alkali- oder Erdalkalihydroxid neutralisiert und während der mehrstündigen Umsetzung im wässrigen Medium bei 80 bis 100 °C der pH-Wert der Lösung durch Zugabe einer Base, vorzugsweise eines Alkalihydroxids, zwischen 8 bis 10 gehalten wird und nach der Quaternierung der pH-Wert mit einer organischen oder anorganischen Säure, auf pH 4,5 bis 6 eingestellt wird.

Das erfindungsgemäße Betain kann zur Formulierung von Wasch- und Reinigungsmitteln sowie in kosmetischen Formulierungen, z.B. in Shampoos, Duschbädern und Flüssigseifen verwendet werden. In Formulierungen kann es kombiniert werden mit z.B.: Netzmittel, Tenside und/oder Emulgatoren der Gruppen anionischer, kationischer, zwitterionischer, amphoterer und/oder nichtionischer Tenside wie Fettalkoholsulfate, Fettalkoholethersulfate, Alkylsulfonate, Alkylbenzolsulfonate, Alkylsulfosuccinate, Alkylethersulfosuccinate, quarternäre Ammoniumsalze, Alkylbetaine, andere Fettsäureamidoalkylbetaine, Derivate von monomeren oder kondensierten Sacchariden wie Zuckerester, Methyl- oder Ethylglucosid-Fettsäureester, Alkylglucoside, ethoxylierte Fettalkohole, Fettsäurealkanolamide, ethoxylierte Fettsäureester, Fettsäuren oder deren Alkali-, Ammonium- Hydroxyalkylammoniumsalze (Seifen) Verdickungsmittel, wie Kaolin, Bentonit, Fettalkohole, Stärke, Polyacrylsäure und Derivate, Cellulose Derivate, Alginate, Vaseline oder Paraffin, weiterhin Trübungsmittel, wie z.B. Derivate von Glycolestern oder Alkohole, wie Ethanol, Propanol, Propylenglykol oder Glycerin, Solubilisatoren, Konsistenzgeber, Puffersysteme, Parfümöle, Farbstoffe, weiterhin Konditionier- und Pflegemittel, wie kationische oder andere amphotere Polymere, Lanolin und Derivative, Cholesterin, Panthenol, Pantothensäure, Betaine, Polydimethylsiloxane und/oder Derivatives, sowie mit allen anderen üblichen kosmetischen Inhaltsstoffen.

### Analysenmethoden:

### Betaingehalt:

Der Betaingehalt wird mittels Titration mit 0,1 M Perchloressigsäure in 1,4-Dioxan bestimmt. Als Lösungsmittel für das Betain wird ein Methanol/Ethylenglycol-monomethylether Gemisch (1 : 3) verwendet, als Elektrode eine pH-Elektrode.

### Wassergehalt:

Der Wassergehalt wird als Differenz aus 100 - Feststoffgehalt in % bestimmt. Er kann auch mittels Karl-Fischer Titration bestimmt werden.

### Feststoffgehalt:

Der Feststoffgehalt wird durch Trocknen des Materials bei 105 °C bis zur Gewichtskonstanz bestimmt.

### Natriumchlorid:

Der Gehalt an Chlorid wird potentiometrisch gegen eine Silbernitrat-Maßlösung ermittelt. Als Elektrode wird eine kombinierte Silberchlorid-Elektrode verwendet.

### Glyceringehalt:

Der Glyceringehalt wird mittels GC nach üblichem Verfahren bestimmt.

### Viskosität:

Die Viskosität wird bei Raumtemperatur mit einem Brookfield-Viskosimeter (Modell LVT) mit Spindel Nr. 3 bei 30 U/min in 150 ml Probe gemessen.

### Beispiele:

### Herstellung des Amidoamins A.1:

Das Amidoamin wurde in bekannter Art und Weise gemäß EP-C-656 346 hergestellt durch Umsetzung von Cocoshartfett und 3-Dimethylaminopropylamin.
In einer 500 ml Rührapparatur mit Rückflusskühler und Stickstoffeinleitung wurden 230 g Cocoshartfett und als Katalysator 15 g Ameisensäure vorgelegt und mit Stickstoff ca. 10 Minuten inertisiert.
Dann werden 180 ml 3-Dimethylaminopropylamin unter Rühren und fortlaufender Inertisierung mit Stickstoff zugegeben. Das Reaktionsgemisch wird auf 165 °C gebracht und etwa 4 bis 5 h bei dieser Temperatur gehalten. Nach dieser Zeit wird überschüssiges DMAPA mittels Vakuumdestillation entfernt.

### Herstellung des Amidoamins A.2:

Das Amidoamin wurde in bekannter Art und Weise gemäß EP-C-656 346 hergestellt durch Umsetzung von Cocoshartfett und 3-Dimethylaminopropylamin.
In einer 500 ml Rührapparatur mit Rückflusskühler und Stickstoffeinleitung wurden 230 g Cocoshartfett und als Katalysator 20 g Essigsäure vorgelegt und mit Stickstoff ca. 10 Minuten inertisiert.
Dann werden 180 ml 3-Dimethylaminopropylamin unter Rühren und fortlaufender Inertisierung mit Stickstoff zugegeben. Das Reaktionsgemisch wird auf 165 °C gebracht und etwa 4 bis 5 h bei dieser Temperatur gehalten. Nach dieser Zeit wird überschüssiges DMAPA mittels Vakuumdestillation entfernt.

### Herstellung des Amidoamins A.3:

Das Amidoamin wurde in bekannter Art und Weise gemäß EP-C-656 346 hergestellt durch Umsetzung von Cocoshartfett und 3-Dimethylaminopropylamin.
In einer 500 ml Rührapparatur mit Rückflusskühler und Stickstoffeinleitung wurden 230 g Cocoshartfett und als Katalysator 24,3 g Propionsäure vorgelegt und mit Stickstoff ca. 10 Minuten inertisiert.
Dann werden 180 ml 3-Dimethylaminopropylamin unter Rühren und fortlaufender Inertisierung mit Stickstoff zugegeben. Das Reaktionsgemisch wird auf 165 °C gebracht und etwa 4 bis 5 h bei dieser Temperatur gehalten. Nach dieser Zeit wird überschüssiges DMAPA mittels Vakuumdestillation entfernt.

### Herstellung des Amidoamins A.4:

Das Amidoamin wurde in bekannter Art und Weise gemäß EP-C-656 346 hergestellt durch Umsetzung von Cocoshartfett und 3-Dimethylaminopropylamin.
In einer 500 ml Rührapparatur mit Rückflusskühler und Stickstoffeinleitung wurden 230 g Cocoshartfett und als Katalysator 24 g Methacrylsäure vorgelegt und mit Stickstoff ca. 10 Minuten inertisiert.
Dann werden 180 ml 3-Dimethylaminopropylamin unter Rühren und fortlaufender Inertisierung mit Stickstoff zugegeben. Das Reaktionsgemisch wird auf 165 °C gebracht und etwa 4 bis 5 h bei dieser Temperatur gehalten. Nach dieser Zeit wird überschüssiges DMAPA mittels Vakuumdestillation entfernt.

### Herstellung des Amidoamins A.5:

Das Amidoamin wurde in bekannter Art und Weise gemäß EP-C-656 346 hergestellt durch Umsetzung von Cocoshartfett und 3-Dimethylaminopropylamin.
In einer 500 ml Rührapparatur mit Rückflusskühler und Stickstoffeinleitung wurden 237 g Cocoshartfett und als Katalysator 13 g Adipinsäure vorgelegt und mit Stickstoff ca. 10 Minuten inertisiert.
Dann werden 180 ml 3-Dimethylaminopropylamin unter Rühren und fortlaufender Inertisierung mit Stickstoff zugegeben. Das Reaktionsgemisch wird auf 165 °C gebracht und etwa 4 bis 5 h bei dieser Temperatur gehalten. Nach dieser Zeit wird überschüssiges DMAPA mittels Vakuumdestillation entfernt.

### Herstellung des Amidoamins B:

Das Amidoamin wurde gemäß Stand der Technik (DE-C-4 207 386) hergestellt durch Umsetzung von Cocoshartfett und 3-Dimethylaminopropylamin in Anwesenheit einer katalysierenden Fettsäure.
In einer 500 ml Rührapparatur mit Rückflusskühler und Stickstoffeinleitung wurden 230 g Cocoshartfett und als Katalysator 2 g Cocosfettsäure vorgelegt und mit Stickstoff ca. 10 Minuten inertisiert.
Dann werden 180 ml 3-Dimethylaminopropylamin unter Rühren und fortlaufender Inertisierung mit Stickstoff zugegeben. Das Reaktionsgemisch wird auf 165 °C gebracht und etwa 4 bis 5 h bei dieser Temperatur gehalten. Nach dieser Zeit wird überschüssiges DMAPA mittels Vakuumdestillation entfernt.

### Herstellung des Amidoamins C:

Das Amidoamin wurde gemäß Stand der Technik hergestellt durch Umsetzung von Cocoshartfett und 3-Dimethylaminopropylamin in Abwesenheit einer katalysierenden Fettsäure.
In einer 500 ml Rührapparatur mit Rückflusskühler und Stickstoffeinleitung wurden 230 g Cocoshartfett mit Stickstoff ca. 10 Minuten inertisiert.
Dann werden 180 ml 3-Dimethylaminopropylamin unter Rühren und fortlaufender Inertisierung mit Stickstoff zugegeben. Das Reaktionsgemisch wird auf 165 °C gebracht und etwa 4 bis 5 h bei dieser Temperatur gehalten. Nach dieser Zeit wird überschüssiges DMAPA mittels Vakuumdestillation entfernt.

### Herstellung einer konzentrierten wässrigen Betainlösung:

### Beispiel 1.1:

### (erfindungsgemäß)

In einem 1l Vierhalskolben, ausgerüstet mit Rührer, Thermometer, Rückflusskühler und Tropftrichter werden 40 g Na-Monochloracetat und 115,6 g Wasser eingewogen.
Nach Zugabe von 1,3 g polyethoxyliertem Rizinusöl mit 200 Oxethyleneinheiten werden über einen Zeitraum von 15 Minuten 100 g Cocosfettamidopropyldimethylamin, wie in Beispiel A.1 beschrieben, zugegeben. Dann erwärmt man das Reaktionsgemisch auf 70 °C und hält die Temperatur bis die Wärmetönung vorüber ist. Dann wird auf 98 °C erhitzt. Nach ca. 7 h liegt der Gehalt an Amid unter 0,5 %.
Es werden 256 g einer wässrigen Betainlösung der folgenden Zusammensetzung erhalten:

| | |
|---|---|
| Betaingehalt: | 47,2 % |
| Wasser: | 45,0 % |
| Viskosität: | 170 mPa·s |

### Beispiel 1.2:

### (erfindungsgemäß)

In einem 1 l Vierhalskolben, ausgerüstet mit Rührer, Thermometer, Rückflusskühler und Tropftrichter werden 40 g Na-Monochloracetat und 115,6 g Wasser eingewogen.
Nach Zugabe von 1,3 g polyethoxyliertem Rizinusöl mit 200 Oxethyleneinheiten werden über einen Zeitraum von 15 Minuten 100 g Cocosfettamidopropyldimethylamin wie in Beispiel A.2 beschrieben, zugegeben. Dann wird das Reaktionsgemisch auf 70 °C erwärmt und die Temperatur bis die Wärmetönung vorüber ist, gehalten. Dann wird auf 98 °C erhitzt. Nach ca. 7 h liegt der Gehalt an Amid unter 0,5 %.
Es werden 256 g einer wässrigen Betainlösung der folgenden Zusammensetzung erhalten:

| | |
|---|---|
| Betaingehalt: | 46,0 % |
| Wasser: | 45,0 % |
| Viskosität: | 280 mPa·s |

### Beispiel 1.3:

### (erfindungsgemäß)

In einem 1 l Vierhalskolben, ausgerüstet mit Rührer, Thermometer, Rückflusskühler und Tropftrichter werden 40 g Na-Monochloracetat und 115,6 g Wasser eingewogen.
Nach Zugabe von 1,3 g polyethoxyliertem Rizinusöl mit 200 Oxethyleneinheiten werden über einen Zeitraum von 15 Minuten 100 g Cocosfettamidopropyldimethylamin, wie in Beispiel A.3 beschrieben, zugegeben. Dann wird das Reaktionsgemisch auf 70 °C erwärmt und die Temperatur bis die Wärmetönung vorüber ist, gehalten. Dann wird auf 98 °C erhitzt. Nach ca. 7 h liegt der Gehalt an Amid unter 0,5 %.
Es werden 256 g einer wässrigen Betainlösung der folgenden Zusammensetzung erhalten:

| | |
|---|---|
| Betaingehalt: | 47,1 % |
| Wasser: | 45,0 % |
| Viskosität: | fest |

### Beispiel 1.4:

### (erfindungsgemäß)

In einem 1 l Vierhalskolben, ausgerüstet mit Rührer, Thermometer, Rückflusskühler und Tropftrichter werden 40 g Na-Monochloracetat und 115,6 g Wasser eingewogen.
Nach Zugabe von 1,3 g polyethoxyliertem Rizinusöl mit 200 Oxethyleneinheiten werden über einen Zeitraum von 15 Minuten 100 g Cocosfettamidopropyldimethylamin, wie in Beispiel A.4 beschrieben, zugegeben. Dann wird das Reaktionsgemisch auf 70 °C erwärmt und die Temperatur bis die Wärmetönung vorüber ist, gehalten. Dann wird auf 98 °C erhitzt. Nach ca. 7 h liegt der Gehalt an Amid unter 0,5 %.
Es werden 256 g einer wässrigen Betainlösung der folgenden Zusammensetzung erhalten:

| | |
|---|---|
| Betaingehalt: | 50,3 % |
| Wasser: | 45,0 % |
| Viskosität: | 100 mPa·s |

### Beispiel 1.5:

### (erfindungsgemäß)

In einem 1 l Vierhalskolben, ausgerüstet mit Rührer, Thermometer, Rückflusskühler und Tropftrichter werden 40 g Na-Monochloracetat und 116 g Wasser eingewogen.
Nach Zugabe von 1,3 g polyethoxyliertem Rizinusöl mit 200 Oxethyleneinheiten werden über einen Zeitraum von 15 Minuten 100 g Cocosfettamidopropyldimethylamin, wie in Beispiel A.5 beschrieben, zugegeben. Dann wird das Reaktionsgemisch auf 70 °C erwärmt und die Temperatur bis die Wärmetönung vorüber ist, gehalten. Dann wird auf 98 °C erhitzt. Nach ca. 7 h liegt der Gehalt an Amid unter 0,5 %.
Es werden 257,3 g einer wässrigen Betainlösung der folgenden Zusammensetzung erhalten:

| | |
|---|---|
| Betaingehalt: | 49,1 % |
| Wasser: | 45,0 % |
| Viskosität: | 170 mPa·s |

### Beispiel 1.6:

### (erfindungsgemäß)

In einem 1 l Vierhalskolben, ausgerüstet mit Rührer, Thermometer, Rückflusskühler und Tropftrichter werden 32,5 g Monochloressigsäure und 106,8 g Wasser eingewogen.
Man fügt 28,9 g 50%iges Natriumhydroxid unter Rühren über den Tropftrichter zu. Die Temperatur steigt dabei auf ca. 50 °C an. Es werden über einen Zeitraum von 15 Minuten 100 g Cocosfettamidopropyldimethylamin, wie in Beispiel A.1 beschrieben, mit einem Gehalt von 1,3 g polyethoxyliertem Rizinusöl mit 200 EO-Einheiten zugefügt. Dann erwärmt man das Reaktionsgemisch auf 70 °C und hält die Temperatur bis die Wärmetönung vorüber ist.
Dann wird auf 98 °C erhitzt. Nach ca. 7 h liegt der Gehalt an Amid unter 0,5 %.
Es werden 269,5 g einer wässrigen Betainlösung der folgenden Zusammensetzung erhalten:

| | |
|---|---|
| Betaingehalt: | 49,0 % |
| Wasser: | 45,0 % |
| Viskosität: | 185 mPa·s |

### Beispiel 1.7:

### (erfindungsgemäß)

In einem 1 l Vierhalskolben, ausgerüstet mit Rührer, Thermometer, Rückflusskühler und Tropftrichter werden 33,2 g Monochloressigsäure und 85,2 g Wasser eingewogen.
Man fügt 29,6 g 50%iges Natriumhydroxid unter Rühren über den Tropftrichter zu. Die Temperatur steigt dabei auf ca. 50 °C an.
Nach Zugabe von 1,8 g polyethoxyliertem Rizinusöl mit 200 Oxethyleneinheiten werden über einen Zeitraum von 15 Minuten 100 g Cocosfettamidopropyldimethylamin, wie in Beispiel A.1 beschrieben, zugegeben. Dann wird das Reaktionsgemisch auf 70 °C erwärmt und die Temperatur bis die Wärmetönung vorüber ist, gehalten. Dann wird auf 98 °C erhitzt. Nach ca. 7 h liegt der Gehalt an Amid unter 0,5 %.
Es werden 249,8 g einer wässrigen Betainlösung mit 60 % Feststoff erhalten, welche sich wie folgt zusammensetzt:

| | |
|---|---|
| Betaingehalt: | 49,2 % |
| Wasser: | 40,0 % |
| Viskosität: | 180 mPa's |

### Beispiel 2.1:

### (nicht erfindungsgemäß)

In einem 1 l Vierhalskolben, ausgerüstet mit Rührer, Thermometer, Rückflusskühler und Tropftrichter werden 38 g Na-Monochloracetat und zunächst 92 g Wasser eingewogen.
Es werden über einen Zeitraum von 15 Minuten 100 g Cocosfettamidopropyldimethylamin, wie in Beispiel A.1 beschrieben, zugegeben. Dann wird das Reaktionsgemisch auf 70 °C erwärmt und die Temperatur bis die Wärmetönung vorüber ist, gehalten. Danach findet eine Verfestigung bzw. Vergelung statt. Das Reaktionsgemisch wurde unrührbar. Durch Verdünnen mit 76,6 g Wasser wird das Reaktionsgemisch wieder mühsam in eine rührbare Form gebracht. Dann wird auf 98 °C erhitzt. Nach ca. 7 h liegt der Gehalt an Cocosfettaminoamid unter 0,5 %.
Es werden 306,7 g einer wässrigen Betainlösung der folgenden Zusammensetzung erhalten:

| | |
|---|---|
| Betaingehalt: | 34,2 % |
| Wasser: | 55,0 % |
| Viskosität: | 125 mPa·s |

### Beispiel 2.2:

### (nicht erfindungsgemäß)

In einem 1 l Vierhalskolben, ausgerüstet mit Rührer, Thermometer, Rückflusskühler und Tropftrichter werden 38 g Na-Monochloracetat und zunächst 92 g Wasser eingewogen.
Es werden über einen Zeitraum von 15 Minuten 100 g Cocosfettamidopropyldimethylamin, wie in Beispiel A.2 beschrieben, zugegeben. Dann wird das Reaktionsgemisch auf 70 °C erwärmt und die Temperatur bis die Wärmetönung vorüber ist, gehalten. Danach findet eine Verfestigung bzw. Vergelung statt. Das Reaktionsgemisch wurde unrührbar. Durch Verdünnen mit 76,6 g Wasser wird das Reaktionsgemisch wieder mühsam in eine rührbare Form gebracht. Dann wird auf 98 °C erhitzt. Nach ca. 7 h liegt der Gehalt an Cocosfetttaminoamid unter 0,5 %.
Es werden 306,7 g einer wässrigen Betainlösung der folgenden Zusammensetzung erhalten:

| | |
|---|---|
| Betaingehalt: | 34,3 % |
| Wasser: | 55,0 % |
| Viskosität: | 445 mPa·s |

### Beispiel 2.3:

### (nicht erfindungsgemäß)

In einem 1 l Vierhalskolben, ausgerüstet mit Rührer, Thermometer, Rückflusskühler und Tropftrichter werden 38 g Na-Monochloracetat und zunächst 92 g Wasser eingewogen.
Es werden über einen Zeitraum von 15 Minuten 100 g Cocosfettamidopropyldimethylamin, wie in Beispiel A.3 beschrieben, zugegeben. Dann wird das Reaktionsgemisch auf 70 °C erwärmt und die Temperatur bis die Wärmetönung vorüber ist, gehalten. Danach findet eine Verfestigung bzw. Vergelung statt. Das Reaktionsgemisch wurde unrührbar. Durch Verdünnen mit 76,6 g Wasser wird das Reaktionsgemisch wieder mühsam in eine rührbare Form gebracht. Dann wird auf 98 °C erhitzt. Nach ca. 7 h liegt der Gehalt an Cocosfetttaminoamid unter 0,5 %.
Es werden 306,7 g einer wässrigen Betainlösung der folgenden Zusammensetzung erhalten:

| | |
|---|---|
| Betaingehalt: | 34,5 % |
| Wasser: | 55,0 % |
| Viskosität: | fest |

### Beispiel 2.4:

### (nicht erfindungsgemäß)

In einem 1 l Vierhalskolben, ausgerüstet mit Rührer, Thermometer, Rückflusskühler und Tropftrichter werden 38 g Na-Monochloracetat und zunächst 92 g Wasser eingewogen.
Es werden über einen Zeitraum von 15 Minuten 100 g Cocosfettamidopropyldimethylamin, wie in Beispiel A.4 beschrieben, zugegeben. Dann wird das Reaktionsgemisch auf 70 °C erwärmt und die Temperatur bis die Wärmetönung vorüber ist, gehalten. Danach findet eine Verfestigung bzw. Vergelung statt. Das Reaktionsgemisch wurde unrührbar. Durch Verdünnen mit 76,6 g Wasser wird das Reaktionsgemisch wieder mühsam in eine rührbare Form gebracht. Dann wird auf 98 °C erhitzt. Nach ca. 7 h liegt der Gehalt an Cocosfetttaminoamid unter 0,5 %.
Es werden 306,8 g einer wässrigen Betainlösung der folgenden Zusammensetzung erhalten:

| | |
|---|---|
| Betaingehalt: | 35,5 % |
| Wasser: | 55,0 % |
| Viskosität: | 100 mPa·s |

### Beispiel 3.1:

### (erfindungsgemäß)

In einem 1 l Vierhalskolben, ausgerüstet mit Rührer, Thermometer, Rückflusskühler und Tropftrichter werden 33,2 g Monochloressigsäure und 141 g Wasser eingewogen.
Man fügt 29,6 g 50%iges Natriumhydroxid unter Rühren über den Tropftrichter zu. Die Temperatur steigt dabei auf ca. 50 °C an. Nach Zugabe von 1,8 g polyethoxyliertem Rizinusöl mit 200 Oxethyleneinheiten werden über einen Zeitraum von 15 Minuten 100 g Cocosfettamidopropyldimethylamin, wie in Beispiel B beschrieben, zugegeben. Dann wird das Reaktionsgemisch auf 70 °C erwärmt und die Temperatur bis die Wärmetönung vorüber ist, gehalten. Dann wird auf 98 °C erhitzt. Nach ca. 7 h liegt der Gehalt an Amid unter 0,5 %.
Es werden 305,6 g einer wässrigen Betainlösung mit 48 % Feststoff erhalten, welche sich wie folgt zusammensetzt:

| | |
|---|---|
| Betaingehalt: | 37,7 % |
| Wasser: | 52,0 % |
| Viskosität: | 180 mPa·s |

### Beispiel 3.2:

### (nicht erfindungsgemäß)

In einem 1 l Vierhalskolben, ausgerüstet mit Rührer, Thermometer, Rückflusskühler und Tropftrichter werden 33,2 g Monochloressigsäure und 99,6 g Wasser eingewogen.
Man fügt 29,6 g 50%iges Natriumhydroxid unter Rühren über den Tropftrichter zu. Die Temperatur steigt dabei auf ca. 50 °C an. Über einen Zeitraum von 15 Minuten werden 100 g Cocosfettamidopropyldimethylamin, wie in Beispiel B beschrieben, zugegeben. Dann wird das Reaktionsgemisch auf 70 °C erwärmt und die Temperatur bis die Wärmetönung vorüber ist, gehalten. In dieser Phase findet eine Verfestigung, bzw. Vergelung statt. Das Reaktionsgemisch wird unrührbar. Eine Verdünnung ist nicht möglich da die zugegebene Wassermenge nicht mehr einrührt. Es kommt daher nicht zur Reaktion und zur Entstehung einer wässrigen Betainlösung.

| | |
|---|---|
| Betaingehalt: | nicht bestimmbar |
| Wasser: | 45,0 % |
| Viskosität: | fest |

### Beispiel 4.1:

### (erfindungsgemäß)

In einem 1 l Vierhalskolben, ausgerüstet mit Rührer, Thermometer, Rückflusskühler und Tropftrichter werden 42 g Na-Monochloracetat und 122,6 g Wasser eingewogen.
Nach Zugabe von 6,7 g L-Glutaminsäure wird auf 50 °C erwärmt und 30 Minuten bei dieser Temperatur gehalten.
Nach Zugabe von 1,3 g polyethoxyliertem Rizinusöl mit 200 Oxethyleneinheiten werden über einen Zeitraum von 15 Minuten 100 g Cocosfettamidopropyldimethylamin, wie in Beispiel C beschrieben, zugegeben. Dann wird das Reaktionsgemisch auf 70 °C erwärmt und die Temperatur bis die Wärmetönung vorüber ist, gehalten. Dann wird auf 98 °C erhitzt. Nach ca. 7 h liegt der Gehalt an Amid unter 0,5 %.
Es werden 272,7 g einer wässrigen Betainlösung der folgenden Zusammensetzung erhalten:

| | |
|---|---|
| Betaingehalt: | 44,9 % |
| Wasser: | 45,0 % |
| Viskosität: | 400 mPa·s |

### Beispiel 4.2:

### (erfindungsgemäß)

In einem 1 l Vierhalskolben, ausgerüstet mit Rührer, Thermometer, Rückflusskühler und Tropftrichter werden 42 g Na-Monochloracetat und 127,6 g Wasser eingewogen.
Nach Zugabe von 1,3 g polyethoxyliertem Rizinusöl mit 200 Oxethyleneinheiten werden über einen Zeitraum von 15 Minuten 100 g Cocosfettamidopropyldimethylamin, wie in Beispiel C beschrieben, zugegeben. Dann wird das Reaktionsgemisch auf 70 °C erwärmt und die Temperatur bis die Wärmetönung vorüber ist, gehalten. Nach Zugabe von 6,9 g L-Glutaminsäure wird auf 98 °C erhitzt. Nach ca. 6,5 h liegt der Gehalt an Amid unter 0,5 %.
Es werden 272,4 g einer wässrigen Betainlösung der folgenden Zusammensetzung erhalten:

| | |
|---|---|
| Betaingehalt: | 41,2 % |
| Wasser: | 46,0 % |
| Viskosität: | 160 mPa·s |

### Beispiel 5:

### (erfindungsgemäß)

In einem 1 l Vierhalskolben, ausgerüstet mit Rührer, Thermometer, Rückflusskühler und Tropftrichter werden 32,5 g Monochloressigsäure und 106,8 g Wasser eingewogen.
Man fügt 28,9 g 50%iges Natriumhydroxid unter Rühren über den Tropftrichter zu. Die Temperatur steigt dabei auf ca. 50 °C an. Es werden über einen Zeitraum von 15 Minuten 100 g Cocosfettamidopropyldimethylamin, wie in Beispiel A.1 beschrieben, mit einem Gehalt von 1,3 g polyethoxyliertem Glycerylpalmitat mit 200 EO-einheiten zugefügt. Dann wird das Reaktionsgemisch auf 70 °C erwärmt und die Temperatur bis die Wärmetönung vorüber ist, gehalten. Dann wird auf 98 °C erhitzt. Nach ca. 7 h liegt der Gehalt an Amid unter 0,5 %.
Es werden 269,5 g einer wässrigen Betainlösung der folgenden Zusammensetzung erhalten:

| | |
|---|---|
| Betaingehalt: | 43,9 % |
| Wasser: | 45,0 % |
| Viskosität: | 205 mPa·s |

### Beispiel 6:

### (erfindungsgemäß)

In einem 1 l Vierhalskolben, ausgerüstet mit Rührer, Thermometer, Rückflusskühler und Tropftrichter werden 32,5 g Monochloressigsäure und 106,8 g Wasser eingewogen.
Man fügt 28,9 g 50%iges Natriumhydroxid unter Rühren über den Tropftrichter zu. Die Temperatur steigt dabei auf ca. 50 °C an. Es werden über einen Zeitraum von 15 Minuten 100 g Cocosfettamidopropyldimethylamin, wie in Beispiel A.1 beschrieben, mit einem Gehalt von 1,3 g polyethoxyliertem Sorbitanlaurat mit 160 EO-Einheiten zugefügt. Dann wird das Reaktionsgemisch auf 70 °C erwärmt und die Temperatur gehalten, bis die Wärmetönung vorüber ist.
Dann wird auf 98 °C erhitzt. Nach ca. 7 h liegt der Gehalt an Amid unter 0,5 %.
Es werden 269,5 g einer wässrigen Betainlösung der folgenden Zusammensetzung erhalten:

| | |
|---|---|
| Betaingehalt: | 43,7 % |
| Wasser: | 45,0 % |
| Viskosität: | 210 mPa·s |

### Beispiel 7:

### (erfindungsgemäß)

In einem 1 l Vierhalskolben, ausgerüstet mit Rührer, Thermometer, Rückflusskühler und Tropftrichter werden 32,5 g Monochloressigsäure und 106,8 g Wasser eingewogen.
Man fügt 28,9 g 50%iges Natriumhydroxid unter Rühren über den Tropftrichter zu. Die Temperatur steigt dabei auf ca. 50 °C an. Es werden über einen Zeitraum von 15 Minuten 100 g Cocosfettamidopropyldimethylamin, wie in Beispiel A.1 beschrieben, mit einem Gehalt von 1,3 g polyethoxyliertem Cocosfett mit 200 EO-Einheiten zugefügt. Dann wird das Reaktionsgemisch auf 70 °C erwärmt und die Temperatur bis die Wärmetönung vorüber ist, gehalten.
Dann wird auf 98 °C erhitzt. Nach ca. 7 h liegt der Gehalt an Amid unter 0,5 %.
Es werden 269,5 g einer wässrigen Betainlösung der folgenden Zusammensetzung erhalten:

| | |
|---|---|
| Betaingehalt: | 42,9 % |
| Wasser: | 46,0 % |
| Viskosität: | 120 mPa·s |

### Beispiel 8:

### (erfindungsgemäß)

In einem 1 l Vierhalskolben, ausgerüstet mit Rührer, Thermometer, Rückflusskühler und Tropftrichter werden 42 g Na-Monochloracetat und 127,6 g Wasser eingewogen.
Nach Zugabe von 1,3 g polyethoxyliertem Rizinusöl mit 200 Oxethyleneinheiten werden über einen Zeitraum von 15 Minuten 100 g Cocosfettamidopropyldimethylamin, wie in Beispiel C beschrieben, zugegeben. Dann wird das Reaktionsgemisch auf 70 °C erwärmt und die Temperatur bis die Wärmetönung vorüber ist, gehalten. Nach Zugabe von 15 g Zitronensäure wird auf 98 °C erhitzt. Nach ca. 6,5 h liegt der Gehalt an Amid unter 0,5 %.
Es werden 272,4 g einer wässrigen Betainlösung der folgenden Zusammensetzung erhalten:

| | |
|---|---|
| Betaingehalt: | 44,2 % |
| Wasser: | 45,0 % |
| Viskosität: | 120 mPa·s |

### Anwendungsbeispiel A :

### Konzentrierte Geschirrspülmittel

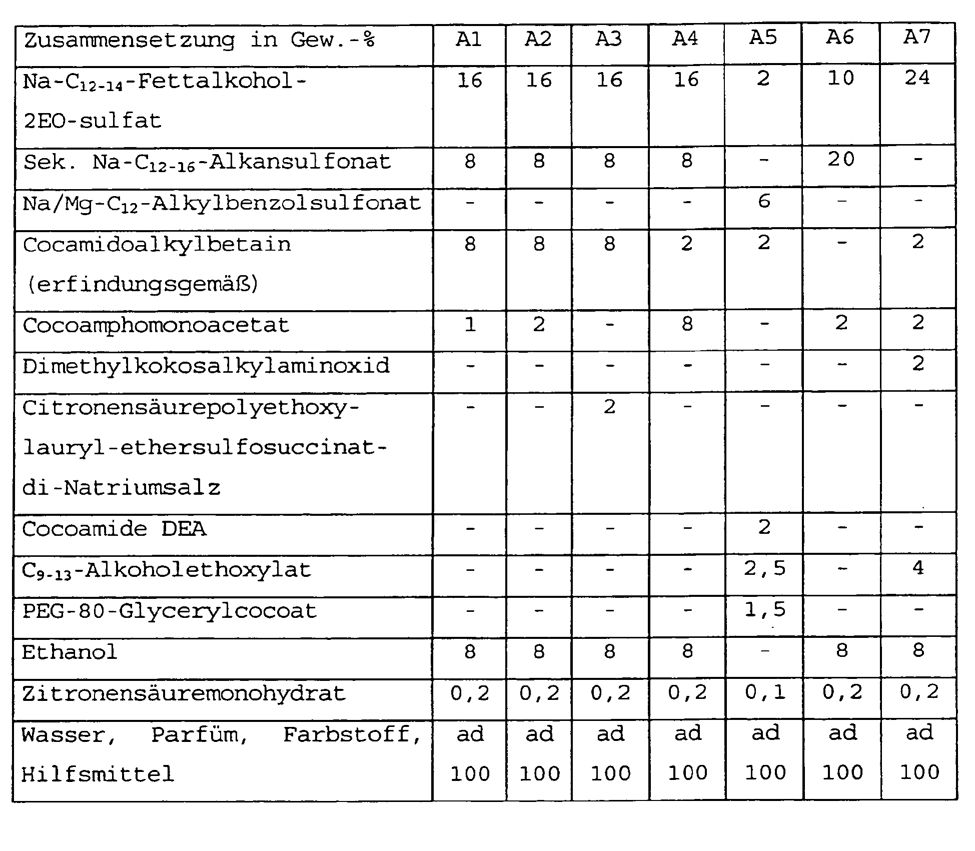

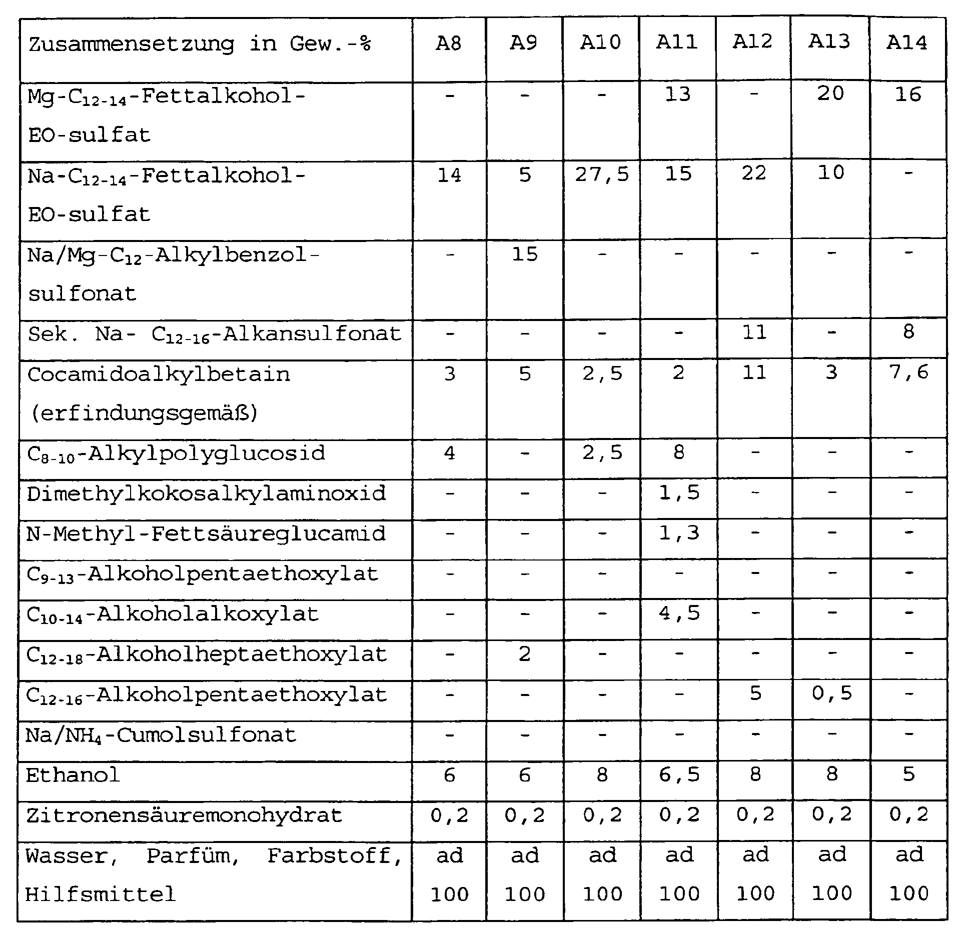

### Reduktion des Alkoholgehaltes durch das erfindungsgemäß hergestellte Cocoamidoalkylbetain

### Unterschiedlich hergestellte Betaine in Formulierung A14

| Zusammensetzung CAPB* in A 14 | Viskosität 4 %-EtOH | Viskosität 5 %-EtOH | Viskosität 6 %-EtOH |
|---|---|---|---|
| Marktprodukt Tego^{®} Betain F50 | | 800 | 460 |
| Bsp. 1.4 | 860 | 450 | |
| Bsp. 4.1 | | 530 | |
| Bsp. 1.1 | | 410 | |
| Bsp. 1.2 | | 440 | |
| Bsp. 4.2 | | 420 | |

| | | | |
|---|---|---|---|
| *Cocoamidopropylbetain | | | |

In handelsüblichen Geschirrspülmittelformulierungen sind in der Regel ≥ 5 Gew.-% Ethanol erforderlich, um die Formulierung fließfähig zu machen und zu halten. Wie aus der Tabelle ersichtlich, können durch das erfindungsgemäße Cocamidoalkylbetain ca. 20 % des benötigten Alkohols eingespart werden.

In den Beispielen A1 bis A13 wurden vergleichbare Ergebnisse erhalten.

## Patentansprüche

1. Verfahren zur Herstellung von hochkonzentrierten fließ- und pumpfähigen wässrigen, d.h. vorzugsweise von organisch lösungsmittelfreien Lösungen von Betainen der allgemeinen Formel (III)
R³-CONH-(CH₂)ₘ-N⁺R⁴R⁵(CH₂)_{y}COO⁻ (III)
worin
R³ der Alkylrest einer Fettsäure ist, der gegebenenfalls verzweigt, gegebenenfalls Mehrfachbindungen, gegebenenfalls Hydroxylgruppen enthalten kann,
R⁴, R⁵ gleiche oder verschiedene Alkylreste mit 1 bis 4 C-Atomen bedeuten,
m 1 bis 3 und
y 1, 2, 3 sein kann,
mit einem Betaingehalt von mindestens 32 Gew.-% durch Quaternierung von tertiären Aminstickstoff enthaltenden Verbindungen mit ω-Halogencarbonsäuren nach bekannten Verfahren, **dadurch gekennzeichnet, dass** der Reaktionsmischung vor oder während der Quaternierungsreaktion 0,1 bis < 3 Gew.-%, vorzugsweise 0,1 bis 1 Gew.-%, bezogen auf das Endprodukt, eines oder mehrerer mizellarer Verdicker zugesetzt werden.

2. Verfahren zur Herstellung von hochkonzentrierten fließ- und pumpfähigen wässrigen Lösungen von Betainen gemäß Anspruch 1, **dadurch gekennzeichnet, dass** der Reaktionsmischung vor oder während der Quaternierungsreaktion als mizellare Verdicker 0,1 bis < 3 Gew.-%, vorzugsweise 0,1 bis 1 Gew.-%, bezogen auf das Endprodukt, eines oder mehrerer nichtionogener Tenside zugesetzt werden.

3. Verfahren zur Herstellung von hochkonzentrierten fließ- und pumpfähigen wässrigen Lösungen von Betainen gemäß den Ansprüchen 1 und/oder 2, **dadurch gekennzeichnet, dass** als mizellare Verdicker vor oder während der Quaternierungsreaktion 0,1 bis < 3 Gew.-%, vorzugsweise 0,1 bis 1 Gew.-%, bezogen auf das Endprodukt, eines oder mehrerer nichtionogener Tenside mit einem HLB-Wert > 12 zugesetzt werden.

4. Verfahren nach mindestens einem der Ansprüche 1 bis 3, **dadurch gekennzeichnet, dass** als mizellare Verdicker 0,1 bis < 3 Gew.-%, vorzugsweise 0,1 bis 1 Gew.-%, bezogen auf das Endprodukt, ein oder mehrere Polyoxyethylenether und/oder -ester einer Hydroxyfettsäure bzw. eines Hydroxyfettsäureglycerides eingesetzt werden.

5. Verfahren nach mindestens einem der Ansprüche 1 bis 4, **dadurch gekennzeichnet, dass** als viskositätsreduzierende Mittel vor, während oder nach der Quaternierungsreaktion zusätzlich mindestens eine Verbindung ausgesucht aus der Gruppe der Sulfobetaine, Amphoglycinate, Trimethylglycin, Aminosäuren; N-Betaine auf Basis von Aminosäuren wie ein- oder mehrwertigen, gegebenenfalls Mehrfachbindungen und/oder OH-Gruppen enthaltenden C₁₋₆-Carbonsäureamidobetaine insbesondere C₁₋₃-Carbonsäureamidopropylbetaine; Monocarbonsäureamide, Monocarbonsäureamidoamine, Di-/Polycarbonsäuremono- und/oder -diamide, einwertigen, gegebenenfalls hydroxyfunktionalisierten Carbonsäuren, di-/polyfunktionellen, gegebenenfalls hydroxyfunktionalisierten Carbonsäuren, wasserlöslichen Kohlenhydraten, Polyole mitverwendet werden.

6. Verfahren nach mindestens einem der Ansprüche 1 bis 4, **dadurch gekennzeichnet, dass** die Amidierung der ersten Stufe in Gegenwart von mindestens einer ein- oder mehrwertigen, gegebenenfalls Mehrfachbindungen und/oder OH-Gruppen und/oder Amino-Gruppen enthaltenden Carbonsäuren durchgeführt wird.

7. Hochkonzentrierte fließ- und pumpfähige wässrige Lösungen von Betainen mit einem Betaingehalt von mindestens 32 Gew.-%, hergestellt nach mindestens einem der Ansprüche 1 bis 6.

8. Hochkonzentrierte fließ- und pumpfähige wässrige Lösungen von Betainen der allgemeinen Formel (III)
R³-CONH-(CH₂)ₘ-N⁺R⁴R⁵(CH₂)_{y}COO⁻ (III)
worin
R³ der Alkylrest einer Fettsäure ist, der gegebenenfalls verzweigt, gegebenenfalls Mehrfachbindungen, gegebenenfalls Hydroxylgruppen enthalten kann,
R⁴, R⁵ gleiche oder verschiedene Alkylreste mit 1 bis 4 C-Atomen bedeuten,
m 1 bis 3 und
y 1, 2, 3 sein kann,
mit einem Betaingehalt von mindestens 32 Gew.-%, **dadurch gekennzeichnet, dass** sie einen Gehalt von 0,1 bis < 3 Gew.-%, an mizellaren Verdickern enthalten.

9. Hochkonzentrierte fließ- und pumpfähige wässrige Lösungen von Betainen gemäß Anspruch 8, **dadurch gekennzeichnet, dass** sie zusätzlich mindestens eine Verbindung ausgesucht aus der Gruppe der Sulfobetaine, Amphoglycinate, Trimethylglycin, Aminosäuren; N-Betaine auf Basis von Aminosäuren wie ein- oder mehrwertigen, gegebenenfalls Mehrfachbindungen und/oder OH-Gruppen enthaltenden C₁₋₆-Carbonsäureamidobetaine insbesondere C₁₋₃-Carbonsäureamidopropylbetaine; Monocarbonsäureamide, Monocarbonsäureamidoamine, Di-/Polycarbonsäuremono- und/oder -diamide, einwertigen, gegebenenfalls hydroxyfunktionalisierten Carbonsäuren, di-/polyfunktionellen, gegebenenfalls hydroxyfunktionalisierten Carbonsäuren, wasserlöslichen Kohlenhydraten, Polyole enthalten.

10. Verwendung der Betainkonzentrate gemäß Ansprüchen 7-9 zur Herstellung von Wasch- und Reinigungsmitteln für Haushalt, Industrie und Kosmetik.

## Claims

1. Process for the preparation of highly concentrated flowable and pumpable aqueous, i.e. preferably of organic solvent-free, solutions of betaines of the general formula (III)
R³-CONH-(CH₂)ₘ-N⁺R⁴R⁵(CH₂)_{y}COO⁻ (III)
in which
R³ is the alkyl radical of a fatty acid which optionally branched, can optionally comprise multiple bonds, optionally hydroxyl groups,
R⁴, R⁵ are identical or different alkyl radicals having 1 to 4 carbon atoms,
m may be 1 to 3 and
y may be 1, 2, 3,
with a betaine content of at least 32% by weight by quaternization of compounds containing tertiary amine nitrogen with ω-halocarboxylic acids by known processes, **characterized in that** 0.1 to < 3% by weight, preferably 0.1 to 1% by weight, based on the end product, of one or more micellar thickeners are added to the reaction mixture before or during the quaternization reaction.

2. Process for the preparation of highly concentrated flowable and pumpable aqueous solutions of betaines according to Claim 1, **characterized in that** 0.1 to < 3% by weight, preferably 0.1 to 1% by weight, based on the end product, of one or more nonionic surfactants are added as micellar thickeners to the reaction mixture before or during the quaternization reaction.

3. Process for the preparation of highly concentrated flowable and pumpable aqueous solutions of betaines according to Claims 1 and/or 2, **characterized in that** 0.1 to < 3% by weight, preferably 0.1 to 1% by weight, based on the end product, of one or more nonionic surfactants with an HLB value of > 12 are added as micellar thickeners before or during the quaternization reaction.

4. Process according to at least one of Claims 1 to 3, **characterized in that** 0.1 to < 3% by weight, preferably 0.1 to 1% by weight, based on the end product, of one or more polyoxyethylene ethers and/or esters of a hydroxy fatty acid or of a hydroxy fatty acid glyceride are used as micellar thickeners.

5. Process according to at least one of Claims 1 to 4, **characterized in that** additionally at least one compound chosen from the group of sulfobetaines, amphoglycinates, trimethylglycine, amino acids; N-betaines based on amino acids, such as mono- or polyfunctional C₁₋₆-carboxamidobetaines optionally containing multiple bonds and/or OH groups, in particular C₁₋₃-carboxamidopropylbetaines; monocarboxamides, monocarboxamidoamines, di-/polycarboxylic acid mono- and/or diamides, monobasic, optionally hydroxy-functionalized carboxylic acids, di-/polyfunctional, optionally hydroxy-functionalized carboxylic acids, water-soluble carbohydrates, polyols are co-used as viscosity-reducing agents before, during or after the quaternization reaction.

6. Process according to at least one of Claims 1 to 4, **characterized in that** the amidation of the first stage is carried out in the presence of at least one mono- or polybasic carboxylic acid optionally containing multiple bonds and/or OH groups and/or amino groups.

7. Highly concentrated flowable and pumpable aqueous solutions of betaines with a betaine content of at least 32% by weight prepared according to at least one of Claims 1 to 6.

8. Highly concentrated flowable and pumpable aqueous solutions of betaines of the general formula (III)
R³-CONH-(CH₂)ₘ-N⁺R⁴R⁵(CH₂)_{y}COO⁻ (III)
in which
R³ is the alkyl radical of a fatty acid which optionally branched, can optionally comprise multiple bonds, optionally hydroxyl groups,
R⁴, R⁵ are identical or different alkyl radicals having 1 to 4 carbon atoms,
m may be 1 to 3 and
y may be 1, 2, 3,
with a betaine content of at least 32% by weight, **characterized in that** they have a content of from 0.1 to < 3% by weight of micellar thickeners.

9. Highly concentrated flowable and pumpable aqueous solutions of betaines according to Claim 8, **characterized in that** they additionally comprise at least one compound chosen from the group of sulfobetaines, amphoglycinates, trimethylglycine, amino acids; N-betaines based on amino acids, such as mono- or polyfunctional C₁₋₆-carboxamidobetaines optionally containing multiple bonds and/or OH groups, in particular C₁₋₃-carboxamidopropylbetaines ; monocarboxamides, monocarboxamidoamines, di-/polycarboxylic mono- and/or diamides, monobasic, optionally hydroxy-functionalized carboxylic acids, di-/polyfunctional, optionally hydroxy-functionalized carboxylic acids, water-soluble carbohydrates, polyols.

10. Use of the betaine concentrates according to Claims 7-9 for the preparation of washing and cleaning compositions for domestic use, industry and cosmetics.

## Revendications

1. Procédé en vue de la préparation de solutions aqueuses hautement concentrées capables de subir un écoulement et une opération de pompage, c'est-à-dire, de préférence, exemptes de solvants organiques, de bétaïnes de la formule générale (III)
R³-CONH-(CH₂)ₘ-N⁺R⁴R⁵(CH₂)_{γ}COO⁻ (III)
où
R³ est le radical alkyle d'un acide gras, le cas échéant, ramifié, qui peut contenir, le cas échéant, des liaisons multiples, le cas échéant, des groupements hydroxyle,
R⁴, R⁵ désignent des radicaux alkyle, identiques ou différents, ayant de 1 à 4 atomes de C,
m peut aller de 1 à 3 et
γ peut être 1, 2, 3,
avec une teneur en bétaïne d'au moins 32% en poids par quaternisation de composés contenant un azote d'amine tertiaire avec des acides ω-halogénocarboxyliques conformément à des procédés connus, **caractérisé en ce que** l'on ajoute au mélange réactionnel, avant ou pendant la réaction de quaternisation, de 0,1 à < 3 % en poids, de préférence, de 0,1 à 1 % en poids, par rapport au produit final, d'un ou de plusieurs épaississants micellaires.

2. Procédé en vue de la préparation de solutions aqueuses hautement concentrées, capables de subir un écoulement et une opération de pompage, de bétaïnes selon la revendication 1, **caractérisé en ce que** l'on ajoute au mélange réactionnel, avant ou pendant la réaction de quaternisation, en tant qu'épaississant micellaire, de 0,1 à < 3 % en poids, de préférence, de 0,1 à 1 % en poids, par rapport au produit final, d'un ou de plusieurs agents tensioactifs non ionogènes.

3. Procédé en vue de la préparation de solutions aqueuses hautement concentrées, capables de subir un écoulement et une opération de pompage, de bétaïnes selon la revendication 1 et/ou la revendication 2, **caractérisé en ce que** l'on ajoute, en tant qu'épaississant micellaire, avant ou pendant la réaction de quaternisation, de 0,1 à < 3 % en poids, de préférence, de 0,1 à 1 % en poids, par rapport au produit final, d'un ou de plusieurs agents tensioactifs non ionogènes ayant une valeur HLB > 12.

4. Procédé selon au moins l'une quelconque des revendications 1 à 3, **caractérisé en ce que** l'on utilise, en tant qu'épaississant micellaire, de 0,1 à < 3 % en poids, de préférence, de 0,1 à 1 % en poids, par rapport au produit final, un ou plusieurs éthers et/ou esters de polyoxyéthylène d'un acide gras hydroxy ou d'un glycéride d'acide gras hydroxy.

5. Procédé selon au moins l'une quelconque des revendications 1 à 4, **caractérisé en ce que** l'on utilise, en tant qu'agent de réduction de la viscosité, avant, pendant ou après la réaction de quaternisation en sus au moins un composé sélectionné parmi le groupe des sulfobétaïnes, de l'amphoglycinate, de la triméthylglycine, des aminoacides ; des N-bétaïnes à base d'aminoacides comme des amidobétaïnes d'acide C₁₋₆-carboxylique monovalentes ou polyvalentes, contenant, le cas échéant, des liaisons multiples et/ou des groupements OH, en particulier des amidopropylbétaïnes d'acide C₁₋₃-carboxylique; des amides d'acide monocarboxylique, des amidoamines d'acide monocarboxylique, des monoamides et/ou des diamides de diacides/de polyacides carboxyliques, des acides carboxyliques monovalents, le cas échéant, hydroxy-fonctionnalisés, des acides carboxyliques difonctionnels/polyfonctionnels, le cas échéant, hydroxy-fonctionnalisés, des hydrates de carbone hydrosolubles, des polyols.

6. Procédé selon l'une quelconque des revendications 1 à 4, **caractérisé en ce que** la formation d'amides de la première étape est effectuée en présence d'au moins un acide carboxylique monovalent ou polyvalent, contenant, le cas échéant, des liaisons multiples et/ou des groupements OH et/ou des groupements amino.

7. Solutions aqueuses fortement concentrées, capables de subir un écoulement et une opération de pompage, de bétaïnes ayant une teneur en bétaïne d'au moins 32 % en poids, préparées selon au moins l'une quelconque des revendications 1 à 6.

8. Solutions aqueuses fortement concentrées, capables de subir un écoulement et une opération de pompage, de bétaïnes de la formule générale (III)
R³-CONH-(CH₂)ₘ-N⁺R⁴R⁵(CH₂)_{γ}COO⁻ (III)
où
R³ est le radical alkyle d'un acide gras, le cas échéant, ramifié, qui peut contenir, le cas échéant, des liaisons multiples, le cas échéant, des groupements hydroxyle,
R⁴, R⁵ désignent des radicaux alkyle, identiques ou différents, ayant de 1 à 4 atomes de C,
m peut aller de 1 à 3 et
γ peut être 1, 2, 3,
avec une teneur en bétaïne d'au moins 32 % en poids, **caractérisées en ce qu'**elles contiennent une teneur de 0,1 à < 3 % en poids d'épaississants micellaires.

9. Solutions aqueuses fortement concentrées, capables de subir un écoulement et une opération de pompage, de bétaïnes selon la revendication 8, **caractérisées en ce qu'**elles contiennent en sus au moins un composé sélectionné parmi le groupe des sulfobétaïnes, de l'amphoglycinate, de la triméthylglycine, des aminoacides ; des N-bétaïnes à base d'aminoacides comme des amidobétaïnes d'acide C₁₋₆-carboxylique monovalentes ou polyvalentes, contenant, le cas échéant, des liaisons multiples et/ou des groupements OH, en particulier des amidopropylbétaïnes d'acide C₁₋₃-carboxylique ; des amides d'acide monocarboxylique, des amidoamines d'acide monocarboxylique, des monoamides et/ou des diamides de diacides/de polyacides carboxyliques, des acides carboxyliques monovalents, le cas échéant, hydroxy-fonctionnalisés, des acides carboxyliques difonctionnels/polyfonctionnels, le cas échéant, hydroxy-fonctionnalisés, des hydrates de carbone hydrosolubles, des polyols.

10. Utilisation des concentrés de bétaïne selon les revendications 7 à 9, en vue de la préparation d'agents de lavage et de détergents pour les secteurs ménager, industriel et cosmétique.
